(19) Europäisches Patentamt
European Patent Office
Office européen des brevets

(11) Veröffentlichungsnummer: **0 544 171 B1**

# (12) EUROPÄISCHE PATENTSCHRIFT

(45) Veröffentlichungstag der Patentschrift: **17.11.94**

(51) Int. Cl.$^5$: **A61K 31/55**, C07D 223/20, C07D 405/12

(21) Anmeldenummer: **92119534.3**

(22) Anmeldetag: **16.11.92**

## (54) Verwendung von 5,6-Dihydro-dibenz[b,e]azepin-6,11-dion-11-oximen zur Herstellungvon antiretroviralen Mitteln.

(30) Priorität: **27.11.91 DE 4138853**

(43) Veröffentlichungstag der Anmeldung:
**02.06.93 Patentblatt 93/22**

(45) Bekanntmachung des Hinweises auf die Patenterteilung:
**17.11.94 Patentblatt 94/46**

(84) Benannte Vertragsstaaten:
**AT BE CH DE DK ES FR GB GR IE IT LI LU MC NL PT SE**

(56) Entgegenhaltungen:
**EP-A- 0 419 861**
**EP-A- 0 429 987**
**GB-A- 1 132 516**
**US-A- 3 431 257**

(73) Patentinhaber: **BAYER AG**

**D-51368 Leverkusen (DE)**

(72) Erfinder: **Wild, Hanno, Dr.**
**Am Ausblick 128**
**W-5600 Wuppertal 1 (DE)**
Erfinder: **Roeben, Wolfgang, Dr.**
**Strässchen Siefen 30**
**W-5060 Bergisch Gladbach 2 (DE)**
Erfinder: **Aichinger, Gerd, Dr.**
**In den Birken 51**
**W-5600 Wuppertal 1 (DE)**
Erfinder: **Paessens, Arnold, Dr.**
**Stresemannstrasse 51**
**W-5657 Haan (DE)**
Erfinder: **Petersen-von Gehr, Jörg, Dr.**
**Bärendorferstrasse 74**
**W-4630 Bochum 1 (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft die Verwendung von 5,6-Dihydro-dibenz[b,e]azepin-6,11-dion-11-oximen zur Herstellung von antiretroviralen Mitteln.

Aus der DE 15 45 856 ist ein Verfahren zur Herstellung von basisch substituierten Derivaten des 5,6-Dihydro-dibenz[b,e]azepin-6,11-dion-11-oxims bekannt, wobei dort auch einige wenige Beispiele mit Aminoalkylresten am Oximsauerstoff beschrieben sind.

Ferner sind aus dem US-Patent 34 31 257 einige basisch substituierte 5,6-Dihydro-dibenz[b,e]azepin-6,11-dion-11-oxime mit psychotropischer Wirkung bekannt, wobei die erfindungsgemäßen Verbindung der allgemeinen Formel (I) teilweise vom Wortlaut des Bedeutungsumfanges ($R^1$ = Alkyl) dieser Publikationen erfaßt werden.

Die GB 1.132.516 beschreibt Morphanthridin Derivate, die als Antidepressiva eingesetzt werden. Keines der Zitate enthält einen Hinweis auf die antiretrovirale Wirksamkeit solcher Substanzen.

Die vorliegende Erfindung betrifft die Verwendung von 5,6-Dihydro-dibenz[b,e]azepin-6,11-dion-11-oxime der allgemeinen Formel (I),

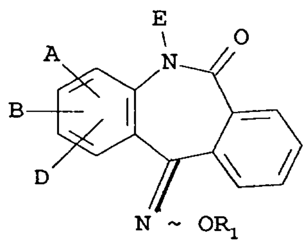

(I)

in welcher

| | |
|---|---|
| A, B und D | gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen, |
| E | für Wasserstoff oder für geradkettiges oder verzweigtes Alkyl mit bis zu 6 Kohlenstoffatomen steht, |
| $R^1$ | für Wasserstoff oder<br>für Cycloalkyl mit 3 bis 6 Kohlenstoffatomen oder für 2-Tetrahydropyranyl steht,<br>für geradkettiges oder verzweigtes Acyl mit bis zu 8 Kohlenstoffatomen steht, oder<br>für geradkettiges oder verzweigtes Alkyl oder Alkenyl mit jeweils bis zu 10 Kohlenstoffatomen steht, die gegebenenfalls durch Halogen, Hydroxy, Carboxy, durch geradkettiges oder verzweigtes Alkoxycarbonyl mit bis zu 6 Kohlenstoffatomen oder durch Phenyl substituiert sind, das seinerseits bis zu 5-fach gleich oder verschieden durch Halogen substituiert sein kann |

gegebenenfalls in einer isomeren Form und deren physiologisch unbedenklichen Salze zur Herstellung von antiretroviralen Mitteln.

Physiologisch unbedenkliche Salze der 5,6-Dihydro-dibenz[b,e]azepin-6,11-dion-11-oxime können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Die Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Verwendung der Antipoden als auch der Racemformen sowie der Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bei dem Rest der allgemeinen Formel (II)

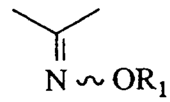

(II)

kann die C=N-Doppelbindung sowohl die E- als auch die Z-Konfiguration besitzen, bzw. es können E/Z-Gemische vorliegen.

Bevorzugt ist die Verwendung von Verbindungen der allgemeinen Formel (I),
in welcher

A, B und D gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen,

E für Wasserstoff oder für geradkettiges oder verzweigtes Alkyl mit bis zu 4 Kohlenstoffatomen steht,

$R^1$ für Wasserstoff oder
für Cyclopropyl, Cyclopentyl, Cyclohexyl oder 2-Tetrahydropyranyl steht, oder
für geradkettiges oder verzweigtes Acyl mit bis zu 6 Kohlenstoffatomen steht, oder
für geradkettiges oder verzweigtes Alkyl oder Alkenyl mit jeweils bis zu 8 Kohlenstoffatomen steht, die gegebenenfalls durch Fluor, Hydroxy, Carboxy, durch geradkettiges oder verzweigtes Alkoxycarbonyl mit bis zu 4 Kohlenstoffatomen oder durch Phenyl substituiert sind, das seinerseits bis zu 5-fach gleich oder verschieden durch Fluor, Chlor oder Brom substituiert sein kann

gegebenenfalls in einer isomeren Form und deren physiologisch unbedenklichen Salze.

Besonders bevorzugt ist die Verwendung von Verbindungen der allgemeinen Formel (I),
in welcher

A, B und D gleich oder verschieden sind und für Wasserstoff, Fluor, Chlor oder für geradkettiges oder verzweigtes Alkyl oder Alkoxy mit jeweils bis zu 4 Kohlenstoffatomen stehen,

E für Wasserstoff, Methyl oder Ethyl steht,

$R^1$ für Wasserstoff oder
für Cyclopropyl oder 2-Tetrahydropyranyl steht, oder
für geradkettiges oder verzweigtes Acyl mit bis zu 6 Kohlenstoffatomen steht, oder
für geradkettiges oder verzweigtes Alkyl oder Alkenyl mit jeweils bis zu 6 Kohlenstoffatomen steht, die gegebenenfalls durch Hydroxy, Carboxy, Fluor, Methoxycarbonyl, Ethoxycarbonyl, Propoxycarbonyl oder durch Phenyl substituiert ist, das seinerseits bis zu 5-fach gleich oder verschieden durch Fluor oder Chlor substituiert sein kann

gegebenenfalls in einer isomeren Form und deren physiologisch unbedenklichen Salze.

Die Verbindungen der allgemeinen Formel (I) können hergestellt werden, indem man

[A] Verbindungen der allgemeinen Formel (III)

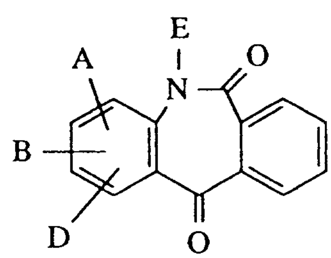

(III)

in welcher
A, B, D und E die oben angegebene Bedeutung haben,
mit Hydroxylaminen der allgemeinen Formel (IV)

$H_2N\text{-}OR^1$ (IV)

in welcher

$R^1$ die oben angegebene Bedeutung hat,
in inerten Lösemitteln, gegebenenfalls in Anwesenheit einer Base umsetzt,
oder
[B] Verbindungen der allgemeinen Formel (Ia)

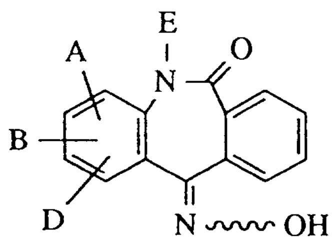

(Ia)

in welcher
A, B, D und E die oben angegebene Bedeutung haben,
mit Verbindungen der allgemeinen Formel (V)

L-$R^2$ (V)

in welcher
$R^2$ die oben angegebene Bedeutung von $R^1$ hat aber nicht für Wasserstoff steht
und
L für eine typische Abgangsgruppe, wie beipsielsweise Tosylat, Mesylat, Chlor, Brom oder Jod steht,
ebenfalls in inerten Losemitteln in Anwesenheit einer Base umsetzt, und gegebenenfalls die Substituenten A, B, D und $R^1$ nach üblichen chemischen Methoden varriiert
und im Fall, daß E nicht Wasserstoff bedeutet, ebenfalls nach bekannten Methoden eine Alkylierung durchführt.

Diese Verfahren können durch folgendes Formelschema bespielhaft erläutert werden:

[A]

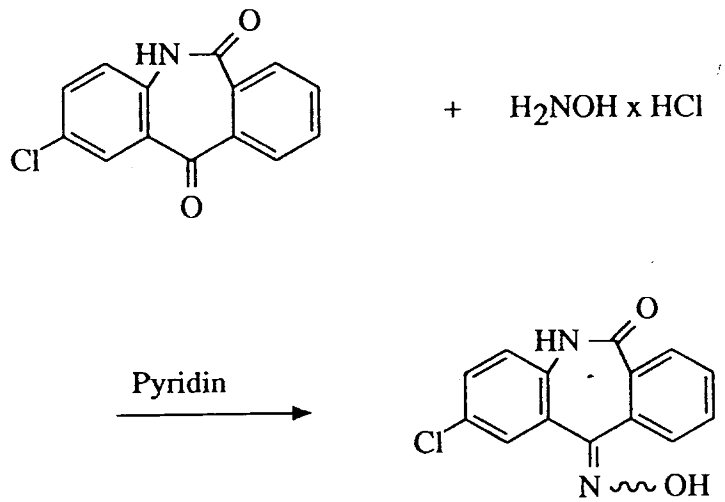


[B]

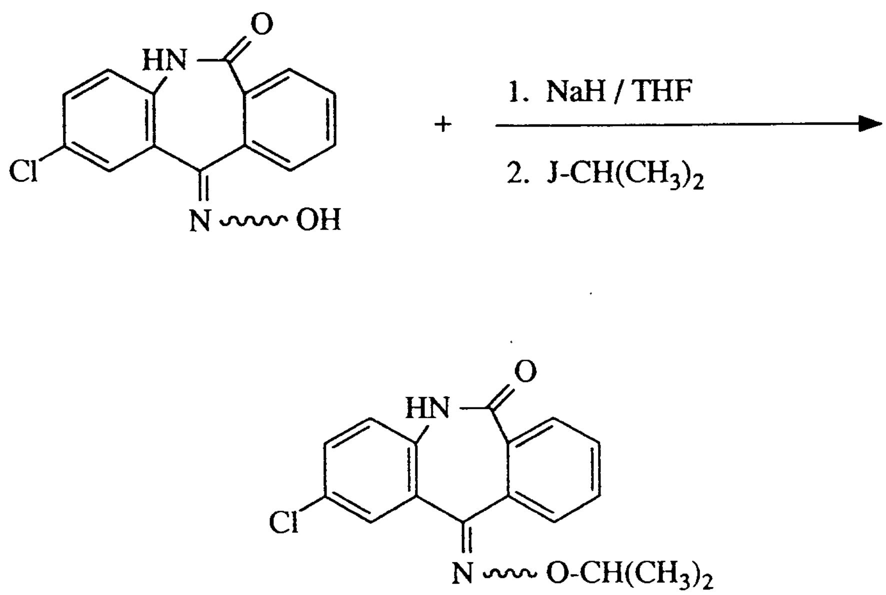


Die oben aufgeführten Verfahren erfolgen in Analogie zu den beschriebenen Methoden aus dem US-Patent 34 31 257.

Als Losemittel eignen sich für die Verfahren [A] und [B] die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Pyridin und Tetrahydrofuran.

Als Basen eignen sich die üblichen basischen Verbindungen. Hierzu gehören vorzugsweise Alkali- oder Erdalkalihydroxide, wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Verfahren [A] und [B] werden im allgemeinen in einem Temperaturbereich von +0°C bis +150°C, bevorzugt von +0°C bis +120°C durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel für die Alkylierung (E ≠ H) eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Aceton.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Verbindungen der allgemeinen Formel (III) sind an sich bekannt oder können nach üblichen Methoden hergestellt werden [vgl. z.B. US 34 31 257].

Auch die Hydroxylamine der allgemeinen Formel (IV) sind bekannt oder können nach bekannten Verfahren hergestellt werden.

Die Verbindungen der allgemeinen Formel (Ia) werden im Fall, daß E ≠ Wasserstoff ist, vom Bedeutungsumfang des US-Patents 34 31 257 erfaßt oder können dann nach dem oben beschriebenen Verfahren [A] hergestellt werden.

Die Verbindungen der allgemeinen Formel (V) sind bekannt [vgl. Beilstein 1, 114].

Die hier beschriebenen Inhibitoren sind Inhibitoren der Reversen Transkriptase und sind als solche für alle Zwecke einsetzbar, für die Enzyminhibitoren geeignet sind. Dies ist zum Beispiel der Einsatz in der Diagnostik um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen enzymatischer Reaktionen verwenden.

Darüber hinaus wurde überraschend gefunden, daß die erfindungsgemäßen Verbindungen der allgemeinen Formel (I) eine außerordentlich starke Wirkung gegen Retroviren besitzen. Sie zeigen Wirkung in Lentivirus-infizierten Zellkulturen. Dies konnte am Beispiel des HIV-Virus gezeigt werden.

<u>HIV-Infektion in Zellkultur</u>

Der HIV-Test wurde mit geringen Modifikationen nach der Methode von Pauwels et al. [vgl. Journal of Virological Methods <u>20</u>, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Alternativ wurden HIV-suszeptible H9-Zellen anstelle von normalen menschlichen Blutlymphozyten zur Testung der antiviralen Effekte der erfindungsgemäßen Verbindungen eingesetzt.

Die Virusadsorptionslösung wurde zentrifugiert und das infizierte Zellpellet in Wachstumsmedium aufgenommen, so daß 1 x $10^5$ Zellen pro ml eingestellt waren. Die derart infizierten Zellen wurden zu 1 x $10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten $2^{10}$fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 - 50 Syncytien, währen die unbehandelte Zellkontrolle keine Syncytien aufwies.

Die $IC_{50}$-Werte wurden als die Konzentration der behandelten und infizierten Zellen ermittelt, bei der 50% (ca. 10 - 20 Syncytien) der virusinduzierten Syncytien durch die Behandlung mit der erfindungsgemäßen Verbindung unterdrückt waren.

Es wurde nun gefunden, daß die hier beschriebenen Verbindungen HIV infizierte Zellen vor der virusinduzierten Zellzerstörung schützen.

Tabelle I

| Bsp.-Nr. | $IC_{50}$ (µM) |
|---|---|
| 4 | 0,06 |
| 6 | 1,5 |
| 8 | 0,5 |
| 17 | 0,7 |
| 18 | 1,0 |
| 24 | 2,3 |
| 31 (Vergleich) | 0,25 |
| BIRG 587 [J. Med. Chem. 34 2231,(1991)] | 0,09 |

Die vorliegenden Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Die Wirkstoffe der Formel (I) sollen in den antiretroviralen Mitteln in einer Konzentration von etwa 0,1 bis 99,5, vorzugsweise von etwa 0,5 bis 95 Gew.-% der Gesamtmischung vorhanden sein.

Die Mittel können außer den Verbindungen der Formel (I) auch weitere pharmazeutische Wirkstoffe enthalten.

Die Herstellung der Mittel erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe in Gesamtmengen von etwa 0,1 bis etwa 200, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthalt den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Herstellungsbeispiele

Beispiel 1

(E/Z)-2-Chlor-11-hydroxyimino-6-oxo-5,6-dihydro-11H-dibenz[b,e]azepin

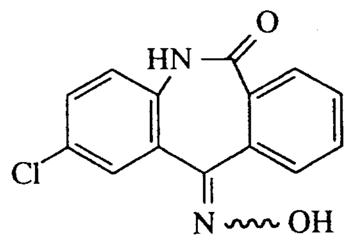

10 g (39 mmol) 2-Chlor-6,11-dioxo-5,6-dihydro-11H-dibenz[b,e]azepin und 3,24 g (46 mmol) Hydroxylaminhydrochlorid in 80 ml Pyridin werden 18 h auf 100 °C erhitzt. Anschließend gießt man in 2 N Salzsäure und filtriert das ausgefallene Produkt ab. Umkristallisation aus Ethanol liefert 9,23 g des Oxims.

Beispiel 2

(E/Z)-11-tert.Butyloxyimino-2-chlor-6-oxo-5,6-dihydro-11H-dibenz[b,e]azepin

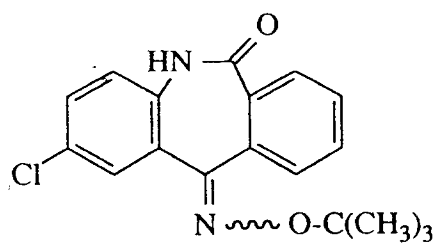

0,5 g (2,2 mmol) 2-Chlor-6,11-dioxo-5,6-dihydro-11H-dibenz[b,e]azepin und 304 mg (2,4 mmol) O-tert.Butylhydroxylamin in 4,4 ml Pyridin werden 6 h auf 100 °C erhitzt. Anschließend gießt man in 2 N Salzsäure und filtriert das ausgefallene Produkt ab. Umkristallisation aus Ethanol/Wasser liefert 307 mg Produkt.
$^1$H-NMR (DMSO): δ = 1,30 und 1,31 (2s, 9H); 7,20 und 7,25 (2d, J = 9 Hz, 1H); 7,35 - 7,8 (m, 5H); 7,9 (m, 1H); 10,68 und 10,74 (2s, NH).

Beispiel 3

(E/Z)-2-Chlor-11-isopropyloxyimino-6-oxo-5,6-dihydro-11H-dibenz[b,e]azepin

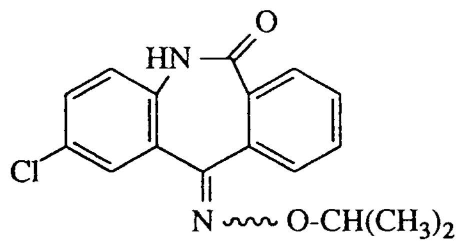

300 mg (1,1 mmol) (E/Z)-2-Chlor-11-hydroxyimino-6-oxo-5,6-dihydro-11H-dibenz[b,e]azepin in 2,2 ml abs. THF werden mit 36,3 mg (1,2 mmol) einer 80%igen Suspension von NaH in Öl versetzt und 30 min unter Rückfluß erhitzt. Es werden 121 µl (1,21 mmol) 2-Iodpropan zugefügt und weitere 18 h unter Rückfluß erhitzt. Anschließend wird filtriert, das Filtrat eingeengt und der Rückstand an Kieselgel mit $CH_2Cl_2$/EtOAc 10:1 gereinigt.
Ausbeute: 125 mg
$^1$H-NMR ($CDCl_3$): $\delta$ = 1,23 und 1,28 (2d, J = 6 Hz, 3H); 1,32 und 1,36 (2d, J = 6 Hz, 3H); 4,50 und 4,52 (2 septett, J = 6 Hz, 1H); 7,05 und 7,10 (2d, J = 9 Hz, 1H); 7,28 (m, 1H); 7,48 - 7,65 (m, 4H); 8,08 (d, J = 9 Hz, 1H); 9,08 und 9,12 (2s, NH).

Beispiel 4

(E)-2-Chlor-11-ethyloxyimino-6-oxo-5,6-dihydro-11H-dibenz[b,e]azepin

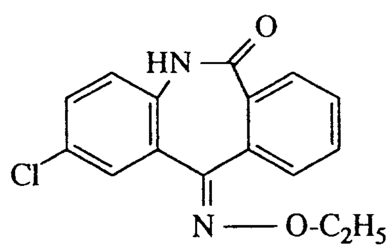

(E/Z)-2-Chlor-11-hydroxyimino-6-oxo-5,6-dihydro-11H-dibenz[b,e]azepin wird durch HPLC an einer Si60-Phase und 3% Isopropanol in Petrolether in die reinen (E)- und (Z)-Komponenten aufgetrennt. 50 mg (0,18 mmol) der (E)-Verbindung in 0,18 ml Ethanol und 0,54 ml THF werden bei 0°C mit 71 mg (0,27 mmol) Triphenylphosphan und dann mit einer Lösung von 56 µl (0,36 mmol) Azodicarbonsäurediethylester in 0,18 ml Ethanol versetzt. Man läßt über Nacht bei Raumtemperatur rühren, dampft ein und reinigt an Kieselgel mit $CH_2Cl_2$/EtOAc 30:1.
Ausbeute: 29 mg
$^1$H-NMR ($CDCl_3$): $\delta$ = 1,30 (t, J = 6 Hz, 3H); 4,26 (m, 2H); 7,00 (d, J = 8 Hz, 1H); 7,30 (m, 1H); 7,45 - 7,70 (m, 4H); 8,08 (d, J = 8 Hz, 1H); 8,61 (s, NH).

In Analogie zu den Vorschriften der Beispiele 1 - 4 werden die in Tabelle 1 aufgeführten Beispiele hergestellt:

Tabelle 1:

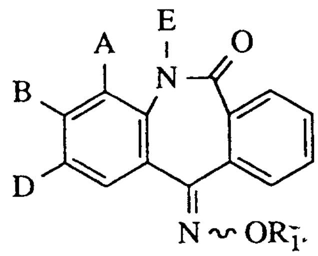

| Bsp.-Nr. | A | B | D | E | $R^1$ | E/Z | analog Beispiel |
|---|---|---|---|---|---|---|---|
| 5 | H | H | H | H | $-CH_3$ | 1:1 | 2 |
| 6 | H | H | -Cl | H | $-CH_3$ | 1:1 | 2 |
| 7 | H | H | -Cl | $-CH_3$ | $-CH_3$ | 1:1 | 2 |
| 8 | H | H | -Cl | H | $-C_2H_5$ | Z | 4 |
| 9 | H | H | H | H | $-C_2H_5$ | 1:1 | 2 |
| 10 | Cl | H | H | H | $-C_2H_5$ | 1:1 | 2 |
| 11 | $-CH_3$ | H | H | H | $-C_2H_5$ | 1:1 | 2 |
| 12 | H | $-CH_3$ | H | H | $-C_2H_5$ | 1:1 | 2 |
| 13 | H | H | $-CH_3$ | H | $-C_2H_5$ | 1:1 | 2 |
| 14 | H | H | -Cl | H | $-C_3H_7$ | 1:1 | 3 |
| 15 | H | H | -Cl | H | 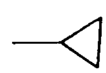 | 1:1 | 2 |
| 16 | H | H | -Cl | H | $-CH_2-CH=CH_2$ | 1:1 | 2 |
| 17 | H | H | -Cl | H | $-CH_2C_6H_5$ | 1:1 | 2 |

Fortsetzung Tabelle 1:

| Bsp.-Nr. | A | B | D | E | $R^1$ | E/Z | analog Beispiel |
|---|---|---|---|---|---|---|---|
| 18 | H | H | -Cl | H | 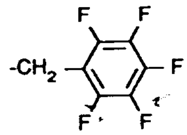 | 1:1 | 2 |
| 19 | H | H | -Cl | H | 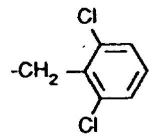 | 1:1 | 2 |
| 20 | H | H | H | H | $-CH_2CO_2H$ | 1:1 | 2 |
| 21 | H | H | -Cl | H | $-CH_2CO_2H$ | 1:1 | 2 |
| 22 | H | H | H | H | $-CH_2CO_2CH_3$ | 1:1 | 2 |
| 23 | H | H | H | H | $-CH_2CO_2CH_3$ | Z | 4 |
| 24 | H | H | -Cl | H | $-CH_2CO_2CH_3$ | 1:1 | 2 |
| 25 | H | H | -Cl | H | $-CH_2CO_2CH_3$ | Z | 4 |
| 26 | H | H | -Cl | $-CH_3$ | $-CH_2CO_2CH_3$ | 1:1 | 2 |
| 27 | H | H | H | H | $-CH_2CO_2C_2H_5$ | 1:1 | 2 |
| 28 | H | H | -Cl | H | $-CH_2-CO_2C_2H_5$ | 1:1 | 2 |
| 29 | H | H | -Cl | $-CH_3$ | $-CH_2-CO_2C_2H_5$ | 1:1 | 2 |
| 30 | H | -H | -Cl | H | $-(CH_2)_4CO_2C_2H_5$ | 1:1 | 2 |
| 31 | H | H | -Cl | H | 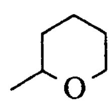 | 1:1 | 2 |
| 32 | H | H | -Cl | H | $-(CH_2)_2OH$ | 1:1 | 2 |
| 33 | H | H | -Cl | H | $-CO-CH_3$ | 1:1 | 2 |
| 34 | H | H | -Cl | H | $-CH\langle^{CH_3}_{CH_3}$ | Z | 4 |
| 35 | H | H | -Cl | H | $-CH\langle^{CH_3}_{CH_3}$ | E | 4 |

## Patentansprüche

1. Verwendung von 5,6-Dihydro-dibenz[b,e]azepin-6,11-dion-11-oximen der allgemeinen Formel (I),

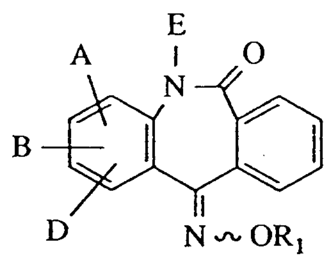

(I)

in welcher

| | |
|---|---|
| A, B und D | gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen, |
| E | für Wasserstoff oder für geradkettiges oder verzweigtes Alkyl mit bis zu 6 Kohlenstoffatomen steht, |
| $R^1$ | für Wasserstoff oder<br>für Cycloalkyl mit 3 bis 6 Kohlenstoffatomen oder für 2-Tetrahydropyranyl steht,<br>für geradkettiges oder verzweigtes Acyl mit bis zu 10 Kohlenstoffatomen steht, oder<br>für geradkettiges oder verzweigtes Alkyl oder Alkenyl mit jeweils bis zu 8 Kohlenstoffatomen steht, die gegebenenfalls durch Halogen, Hydroxy, Carboxy, durch geradkettiges oder verzweigtes Alkoxycarbonyl mit bis zu 6 Kohlenstoffatomen oder durch Phenyl substituiert sind, das seinerseits bis zu 5-fach gleich oder verschieden durch Halogen substituiert sein kann |

gegebenenfalls in einer isomeren Form und deren physiologisch unbedenklichen Salze zur Herstellung von antiretroviral wirksamen Arzneimittel.

2. Verwendung von Verbindungen der allgemeinen Formel (I), gemäß Anspruch 1,
in welcher

| | |
|---|---|
| A, B und D | gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen, |
| E | für Wasserstoff oder für geradkettiges oder verzweigtes Alkyl mit bis zu 4 Kohlenstoffatomen steht, |
| $R^1$ | für Wasserstoff oder<br>für Cyclopropyl, Cyclopentyl, Cyclohexyl oder 2-Tetrahydropyranyl steht, oder<br>für geradkettiges oder verzweigtes Acyl mit bis zu 6 Kohlenstoffatomen steht, oder<br>für geradkettiges oder verzweigtes Alkyl oder Alkenyl mit jeweils bis zu 8 Kohlenstoffatomen steht, die gegebenenfalls durch Fluor, Hydroxy, Carboxy, durch geradkettiges oder verzweigtes Alkoxycarbonyl mit bis zu 4 Kohlenstoffatomen oder durch Phenyl substituiert sind, das seinerseits bis zu 5-fach gleich oder verschieden durch Fluor, Chlor oder Brom substituiert sein kann |

gegebenenfalls in einer isomeren Form und deren physiologisch unbedenklichen Salze zur Herstellung von antiretroviral wirksamen Arzneimitteln.

3. Verwendung von Verbindungen der allgemeinen Formel (I), gemäß Anspruch 1,
in welcher

| | |
|---|---|
| A, B und D | gleich oder verschieden sind und für Wasserstoff, Fluor, Chlor oder für geradkettiges oder verzweigtes Alkyl oder Alkoxy mit jeweils bis zu 4 Kohlenstoffatomen stehen, |
| E | für Wasserstoff, Methyl oder Ethyl steht, |
| $R^1$ | für Wasserstoff oder<br>für Cyclopropyl oder 2-Tetrahydropyranyl steht, oder<br>für geradkettiges oder verzweigtes Acyl mit bis zu 4 Kohlenstoffatomen steht, oder<br>für geradkettiges oder verzweigtes Alkyl oder Alkenyl mit jeweils bis zu 6 Kohlen- |

stoffatomen steht, die gegebenenfalls durch Hydroxy, Carboxy, Fluor, Methoxycarbonyl, Ethoxycarbonyl, Propoxycarbonyl oder durch Phenyl substituiert ist, das seinerseits bis zu 5-fach gleich oder verschieden durch Fluor oder Chlor substituiert sein kann

gegebenenfalls in einer isomeren Form und deren physiologisch unbedenklichen Salze zur Herstellung von antiretroviral wirksamen Arzneimitteln.

**4.** Verwendung gemäß den Ansprüchen 1 - 3, wobei die antiretrovirale Wirkung gegen HIV gerichtet ist.

## Claims

**1.** Use of 5,6-dihydro-dibenz[b,e]azepine-6,11-dione-11-oximes of the general formula (I)

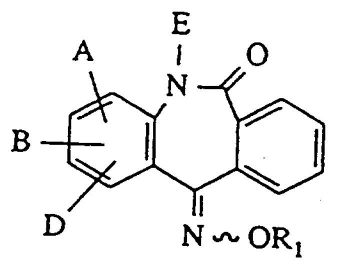

(I)

in which

| | |
|---|---|
| A, B and D | are identical or different and represent hydrogen, amino, nitro, halogen, cyano, hydroxyl, trifluoromethyl, trifluoromethoxy or straight-chain or branched alkyl or alkoxy each having up to 8 carbon atoms, |
| E | represents hydrogen or straight-chain or branched alkyl having up to 6 carbon atoms, |
| $R^1$ | represents hydrogen or<br>represents cycloalkyl having 3 to 6 carbon atoms or 2-tetrahydropyranyl,<br>represents straight-chain or branched acyl having up to 8 carbon atoms, or<br>represents straight-chain or branched alkyl or alkenyl each having up to 10 carbon atoms, each of which is optionally substituted by halogen, hydroxyl or carboxyl, by straight-chain or branched alkoxycarbonyl having up to 6 carbon atoms or by phenyl which in turn can be substituted up to 5 times by identical or different halogen, |

if appropriate in an isomeric form, and their physiologically acceptable salts for the production of antiretroviral medicaments.

**2.** Use of compounds of the general formula (I) according to Claim 1,
in which

| | |
|---|---|
| A, B and D | are identical or different and represent hydrogen, fluorine, chlorine, hydroxyl, trifluoromethyl, trifluoromethoxy or straight-chain or branched alkyl or alkoxy each having up to 6 carbon atoms, |
| E | represents hydrogen or straight-chain or branched alkyl having up to 4 carbon atoms, |
| $R^1$ | represents hydrogen or<br>represents cyclopropyl, cyclopentyl, cyclohexyl or 2-tetrahydropyranyl, or<br>represents straight-chain or branched acyl having up to 6 carbon atoms, or<br>represents straight-chain or branched alkyl or alkenyl each having up to 8 carbon atoms, each of which can optionally be substituted by fluorine, hydroxyl or carboxyl, by straight-chain or branched alkoxy- carbonyl having up to 4 carbon atoms or by phenyl which in turn can be substituted up to 5 times by identical or different fluorine, chlorine or bromine, |

if appropriate in an isomeric form, and their physiologically acceptable salts for the production of antiretroviral medicaments.

**3.** Use of compounds of the general formula (I) according to Claim 1,
in which

| | |
|---|---|
| A, B and D | are identical or different and represent hydrogen, fluorine, chlorine or straight-chain or |

branched alkyl or alkoxy each having up to 4 carbon atoms,

E represents hydrogen, methyl or ethyl,

$R^1$ represents hydrogen or
represents cyclopropyl or 2-tetrahydropyranyl, or
represents straight-chain or branched acyl having up to 4 carbon atoms, or
represents straight-chain or branched alkyl or alkenyl each having up to 6 carbon atoms, each of which is optionally substituted by hydroxyl, carboxyl, fluorine, methoxycarbonyl, ethoxycarbonyl or propoxycarbonyl or by phenyl which in turn can be substituted up to 5 times by identical or different fluorine or chlorine,

if appropriate in an isomeric form, and their physiologically acceptable salts for the production of antiretroviral medicaments.

**4.** Use according to Claims 1 - 3, the antiretroviral action being directed against HIV.

## Revendications

**1.** Utilisation de 11-oximes de 5,6-dihydrodibenz[b,e]azépine-6,11-dione de formule générale (I)

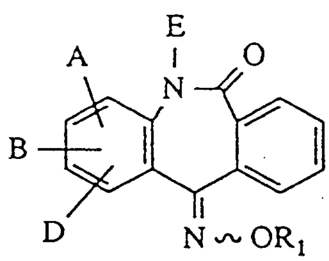

(I)

dans laquelle

A, B et D sont égaux ou différents et représentent de l'hydrogène, un groupe amino, nitro, un halogène, un groupe cyano, hydroxy, trifluorométhyle, trifluorométhoxy ou un groupe alkyle ou alkoxy linéaire ou ramifié ayant chacun jusqu'à 8 atomes de carbone,

E est de l'hydrogène ou un groupe alkyle linéaire ou ramifié ayant jusqu'à 6 atomes de carbone,

$R^1$ est de l'hydrogène ou
un groupe cycloalkyle de 3 à 6 atomes de carbone ou le groupe 2-tétrahydropyrannyle,
un groupe acyle linéaire ou ramifié ayant jusqu'à 10 atomes de carbone, ou bien
un groupe alkyle ou alcényle linéaire ou ramifié ayant chacun jusqu'à 8 atomes de carbone, qui sont substitués le cas échéant par un halogène, un radical hydroxy, carboxy, par un radical alkoxycarbonyle linéaire ou ramifié ayant jusqu'à 6 atomes de carbone ou par un radical phényle qui peut lui-même être substitué jusqu'à 5 fois identiques ou différentes par un halogène,

le cas échéant sous une forme isomère, et de leurs sels acceptables du point de vue physiologique, pour la préparation de médicaments doués d'activité antirétrovirale.

**2.** Utilisation de composés de formule générale (I) suivant la revendication 1,
dans laquelle

A, B et D sont égaux ou différents et représentent de l'hydrogène, du fluor, du chlore, un groupe hydroxy, trifluorométhyle, trifluorométhoxy ou un groupe alkyle ou alkoxy linéaire ou ramifié ayant chacun jusqu'à 6 atomes de carbone,

E est de l'hydrogène ou un groupe alkyle linéaire ou ramifié ayant jusqu'à 4 atomes de carbone,

$R^1$ est de l'hydrogène ou un groupe cyclopropyle, cyclopentyle, cyclohexyle ou 2-tétrahydropyrannyle, ou bien
un groupe acyle linéaire ou ramifié ayant jusqu'à 6 atomes de carbone, ou bien
un groupe alkyle ou alcényle linéaire ou ramifié ayant chacun jusqu'à 8 atomes de carbone, qui sont substitués le cas échéant par du fluor, un radical hydroxy, carboxy,

un radical alkoxycarbonyle linéaire ou ramifié ayant jusqu'à 4 atomes de carbone ou un radical phényle qui peut lui-même être substitué jusqu'à 5 fois identiques ou différentes par du fluor, du chlore ou du brome,

le cas échéant sous une forme isomère, et de leurs sels acceptables du point de vue physiologique pour la préparation de médicaments doués d'activité antirétrovirale.

**3.** Utilisation de composés de formule générale (I) suivant la revendication 1, dans laquelle

A, B et D sont égaux ou différents et représentent de l'hydrogène, du fluor, du chlore, ou un groupe alkyle ou alkoxy linéaire ou ramifié ayant chacun jusqu'à 4 atomes de carbone,

E est de l'hydrogène, ou un groupe méthyle ou éthyle,

$R^1$ est de l'hydrogène ou
un groupe cyclopropyle ou 2-tétrahydropyrannyle, ou bien
un groupe acyle linéaire ou ramifié ayant jusqu'à 4 atomes de carbone, ou bien
un groupe alkyle ou alcényle linéaire ou ramifié ayant chacun jusqu'à 6 atomes de carbone, qui sont substitués le cas échéant par un radical hydroxy, carboxy, fluoro, méthoxycarbonyle, éthoxycarbonyle, propoxycarbonyle ou par un radical phényle qui peut lui-même être substitué jusqu'à 5 fois égales ou différentes par du fluor ou du chlore,

le cas échéant sous une forme isomère, et de leurs sels acceptables du point de vue physiologique pour la préparation de médicaments doués d'activité antirétrovirale.

**4.** Utilisation suivant les revendications 1 à 3, dans laquelle l'activité antirétrovirale est dirigée contre le virus HIV.